Europäisches Patentamt

European Patent Office

Office européen des brevets

(19)

(11) Numéro de publication: **0014166**
**A1**

(12) **DEMANDE DE BREVET EUROPEEN**

(21) Numéro de dépôt: **80420007.9**

(22) Date de dépôt: **18.01.80**

(51) Int. Cl.³: **A 61 M 1/03**

(30) Priorité: **23.01.79 FR 7902575**

(43) Date de publication de la demande: **06.08.80**
**Bulletin 80/16**

(84) Etats contractants désignés: **BE CH DE FR GB IT NL SE**

(71) Demandeur: **SOCIETE DES INDUSTRIES PLASTIQUES - S.O.D.I.P., 7, avenue Lionel Terray, F-69330 Meyzieu (FR)**

(72) Inventeur: **Calvet, Gérard, 9, rue Robespierre, F-94200 Ivry-sur-Seine (FR)**
Inventeur: **Luccioni, Alain, 25, rue du docteur Faguin, F-69500 Bron (FR)**

(74) Mandataire: **Gauckler, Jacques et al, Société HOSPAL - Service Brevets au Centre de Recherches des Carrières de RHONE-POULENC INDUSTRIES, F-69190 Saint-Fons (FR)**

(54) Appareil médical échangeur-séparateur à membranes, son application au traitement du sang et circuit de traitement de sang comprenant un tel appareil.

(57) Appareil à membranes, généralement sélectivement perméables, composé de deux zones distinctes (14, 23) l'une permettant les échanges entre le fluide traité et un fluide auxiliaire à travers une membrane (16), l'autre permettant la séparation d'un composant du fluide traité à travers une membrane (21). Ces zones sont parcourues par le fluide à traiter, soit en parallèle, soit en série.

Application au rein artificiel avec traitement du sang simultané et séparé par hémodialyse et par ultrafiltration.

APPAREIL MEDICAL ECHANGEUR-SEPARATEUR A MEMBRANES

La présente invention concerne un appareil mixte échangeur et séparateur à membrane pour le traitement de fluides en Génie Médical. Elle concerne plus précisément un appareil destiné au traitement du sang par enrichissement, appauvrissement et par échanges de matières ou de calories à l'aide de fluides auxiliaires, liquides ou gazeux, circulant au contact de membranes étanches aux échanges de matières ou de préférence sélectivement perméables. Cet appareil constitue notamment un perfectionnement aux hémodialyseurs, permettant un traitement simultané, mais séparé, du sang par dialyse et par ultrafiltration.

On sait que l'on épure le sang par dialyse contre une solution de liquide de dialyse à travers une membrane semi-perméable par suite des différences de concentration en composés toxiques qui existent entre ces deux liquides. On élimine ainsi progressivement par diffusion à l'aide d'une circulation de liquide de dialyse des substances telles que l'urée, la créatinine et l'acide urique.

On sait également que si la pression exercée par le sang à la surface d'une membrane semi-perméable, comportant généralement des pores de très petits diamètres, est supérieure à la pression existant sur la face opposée de cette membrane, une fraction de l'eau contenue dans le sang, ainsi que certains composés de dimensions inférieures à celles des protéines, traversent les pores de la membrane et sont éliminés par ultrafiltration.

On connait différents types d'hémodialyseurs, équipés de membranes semi-perméables permettant d'effectuer simultanément la dialyse et l'ultrafiltration du sang. Ils nécessitent généralement un appareillage auxiliaire de commande et de contrôle relativement complexe et coûteux pour maitriser avec sécurité et avec une précision suffisante la dialyse et l'ultrafiltration du sang en fonction des caractéristiques du traitement décidées par le médecin pour chaque patient en fonction de son état. En particulier, il est difficile de connaître avec précision le débit et le volume d'ultrafiltrat retiré au patient, généralement inférieur à 4 litres par séance d'hémodialyse, car il se mélange aussitôt aux quelques 300 litres de liquide de dialyse utilisés à chaque traitement, en l'absence de régénération.

.../...

2

D'autre part on a traité des patients en soumettant dans un même hémodialyseur leur sang à des opérations successives dans le temps, d'ultrafiltration et de dialyse, ceci à fin de mieux les maîtriser. Mais on augmente ainsi la durée du traitement si l'on ne veut pas traumatiser les patients.

Enfin, on a effectué des opérations successives dans l'espace d'ultrafiltration et de dialyse du sang, mais en utilisant un ultrafiltre, puis un hémodialyseur reliés en série. Ces deux appareils distincts sont généralement semblables et disposables. Le coût du matériel à usage unique est ainsi généralement augmenté, le raccordement et la mise en oeuvre de deux appareils au lieu d'un seul sont plus longs et plus délicats, en outre le volume de sang hors du corps peut être augmenté.

La présente invention a pour objet un appareil à membrane qui évite les inconvénients des techniques antérieures. Elle a plus précisément pour objet un appareil à membrane qui permette de réaliser à la fois simultanément et séparément des opérations de dialyse et d'ultrafiltration du sang. Elle a pour objet un appareil qui permette un traitement du sang non traumatisant pour le patient, efficace, qui ne nécessite qu'un appareillage annexe de commande et de contrôle simple, sûr et économique. Elle a également pour objet un appareil qui permette d'effectuer à la fois simultanément et séparément des opérations d'échanges et de séparation à l'aide de membranes sur tout fluide, liquide ou gazeux, utilisé en Génie Médical.

Il a maintenant été trouvé et ceci fait l'objet de la présente invention, un appareil pour le traitement de fluides en Génie Médical, comprenant une enveloppe munie d'orifices pour l'introduction et l'évacuation de fluides, caractérisé en ce qu'il comprend au moins une membrane séparant ledit appareil en une première région parcourue par le fluide traité et une deuxième région, ladite deuxième région comportant des moyens la divisant de manière étanche aux fluides en deux compartiments distincts, le premier compartiment étant muni de moyens permettant la circulation d'un fluide auxiliaire et le deuxième compartiment étant muni de moyens permettant l'évacuation d'au moins un composant du fluide traité.

La présente invention concerne un appareil à membrane pour le traitement de fluides en Génie Médical, notamment de sang ou de divers liquides biologiques tels que la lymphe, l'urine, etc ... de liquides utilisés pour le traitement du sang tels que des liquides de dialyse,

.../...

3

des solutions injectables dans le sang, également des gaz tels que l'air, suroxygéné ou non, pour l'oxygénation du sang, le gaz carbonique éliminé du sang, etc ... L'appareil selon l'invention est utilisable pour des traitements effectués avec de tels fluides "in vitro" ou "in vivo".

L'invention sera mieux comprise à l'aide des figures ci-jointes qui illustrent schématiquement et sans échelle déterminée quelques modes de réalisation, donnés à titre d'exemples. Pour plus de commodité, les éléments homologues reçoivent sur les différentes figures les mêmes numéros.

La figure 1 est le schéma d'un appareil selon l'invention comportant deux zones distinctes parcourues en parallèle par le fluide traité.

La figure 2 est le schéma de l'appareil préférentiel selon l'invention comportant deux zones distinctes parcourues en série par le fluide traité.

La figure 3 est le schéma d'un appareil selon l'invention comportant également deux zones distinctes.

La figure 4 est une vue partielle en perspective de deux intercalaires disposés entre les plis en zig-zag d'une membrane.

La figure 5 est une vue en perspective d'un appareil selon l'invention, du type représenté en détails figure 4, constitué par l'empilement d'intercalaires et de membranes.

La figure 6 est la vue en perspective d'un premier mode de réalisation d'un appareil à fibres creuses selon l'invention.

La figure 7 est une vue partielle en coupe d'une variante de réalisation de l'extrémité arrière de l'appareil selon la figure 6.

La figure 8 est la vue en perspective d'un deuxième mode de réalisation d'un appareil à fibres creuses selon l'invention.

La figure 9 est la vue en élévation d'un troisième mode de réalisation d'un appareil à fibres creuses selon l'invention.

La figure 10 est la vue en élévation d'un quatrième mode de réalisation d'un appareil à fibres creuses selon l'invention.

La figure 11 est la vue en élévation d'un cinquième mode de réalisation d'un appareil à fibres creuses selon l'invention.

La figure 12 est le schéma d'un appareil selon l'art antérieur.

La figure 13 est le schéma d'un appareil selon l'invention.

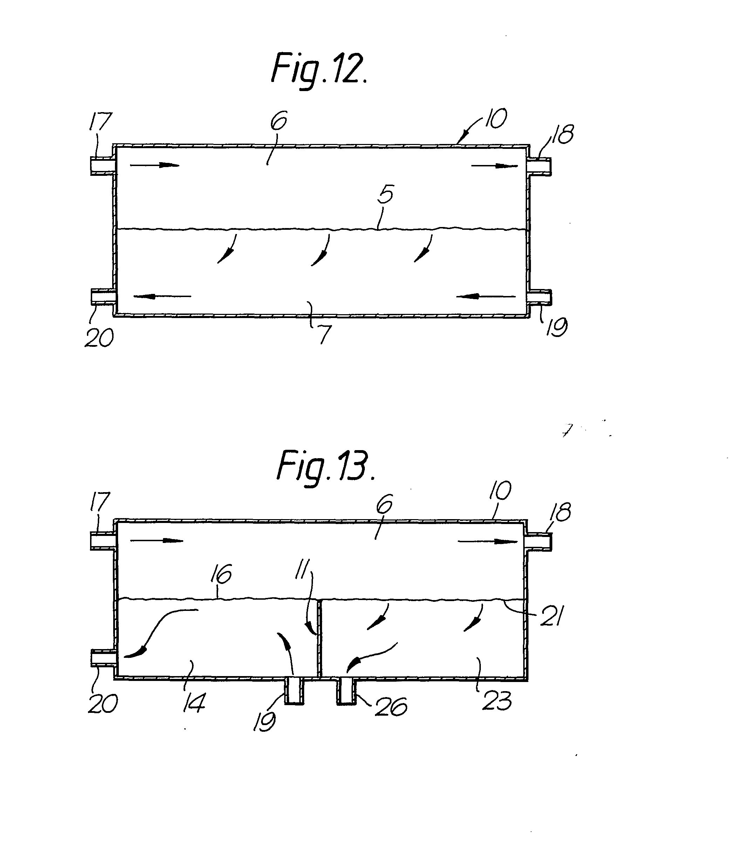

Les appareils à membranes de types connus qui permettent des opérations d'échanges et de séparation de fluides sont représentés schématiquement figure 12. Ils comprennent à l'intérieur d'une enveloppe

.../...

(10) deux régions (6) et (7) séparées par une membrane (5). La première région (6), est parcourue par le fluide traité ; celui-ci pénètre par l'orifice (17) et sort par l'orifice (18). La deuxième région (7), peut être parcourue par un fluide auxiliaire qui pénètre par l'orifice (19). A ce fluide auxiliaire peuvent s'ajouter des composants du fluide traité. Ceux-ci peuvent alors se mélanger au fluide auxiliaire et sortir ensemble par l'orifice (20).

L'appareil selon la présente invention est représenté schématiquement figure 13 ; il comporte des moyens pour séparer de manière étanche aux fluides une telle deuxième région, non parcourue par le fluide traité, en deux compartiments distincts (14) et (23).

Comme moyen on peut employer par exemple une cloison (11), de tout type connu en soi, qui relie de manière étanche aux fluides l'enveloppe (10) à la membrane (16-21). Selon l'invention, seul le premier compartiment (14) est destiné à être parcouru par un fluide auxiliaire pénétrant par l'orifice (19) et sortant par l'orifice (20). La portion (16) de la membrane qui sépare ce premier compartiment (14), de la première région (6) parcourue par le fluide traité assure alors essentiellement une fonction d'échanges de matière et/ou de calories entre le fluide traité et le fluide auxiliaire.

Le deuxième compartiment (23) est destiné à recueillir l'un au moins des composants du fluide traité qui est alors évacué directement par l'orifice (26) sans être mélangé au fluide auxiliaire. La portion (21) de la membrane qui sépare ce deuxième compartiment (23) de la première région (6) parcourue par le fluide à traiter assure une fonction de séparation, par exemple par perméation gazeuse ou par ultrafiltration.

Selon un mode de réalisation particulier de la présente invention, chacun des compartiments de la deuxième région peut à son tour être subdivisé en deux ou plusieurs sous-compartiments, par tous moyens connus, notamment par une cloison s'étendant de manière étanche aux fluides entre l'enveloppe et la membrane. Chacun des sous-compartiments est muni de moyens lui permettant d'assurer séparément une fonction de même nature que le compartiment auquel il appartient. C'est ainsi par exemple qu'un compartiment d'échanges peut être subdivisé en trois sous-compartiments, chacun étant pourvu de moyens permettant la circulation d'un fluide auxiliaire différent. Ainsi le premier sous-compartiment peut être parcouru par un liquide de dialyse pour traiter du sang par hémodialyse, le deuxième sous-compartiment par de l'air

.../...

5

suroxygéné pour assurer l'oxygénation du sang et évacuer le gaz carbonique en excès et le troisième sous-compartiment par un liquide cédant au sang une fraction de ses calories afin de compenser les pertes thermiques de la circulation extracorporelle.

Selon un autre mode de réalisation de la présente invention, la première région peut à son tour être divisée par une deuxième cloison étanche aux fluides en deux nouveaux compartiments situés de préférence en regard des compartiments précédents. Deux cloisons et la membrane séparent alors l'appareil en quatre compartiments distincts.

Le fluide traité peut être divisé à l'entrée de l'appareil en deux fractions dirigées "en parallèle", l'une sur la membrane d'échanges, l'autre sur la membrane de séparation.

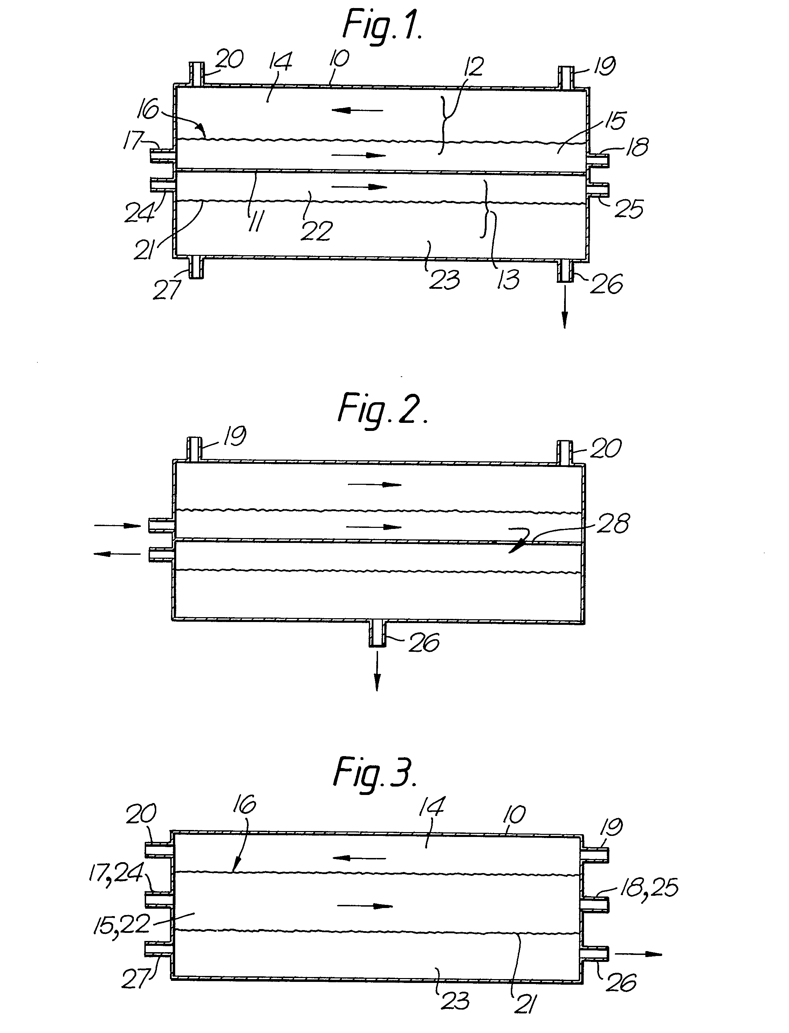

Toutefois on préfère associer à l'appareil des moyens, soit solidaires de l'appareil, internes (orifice (28) figure 2) ou externes (latéraux), soit reliés à celui-ci, pour que le fluide traité circule "en série" d'un compartiment à l'autre dans la première région. Ces moyens peuvent être constitués de canalisations de tous types connus en soi. Cette disposition est préférée parce qu'elle procure une meilleure maîtrise des conditions de vitesse et de pression du fluide traité au contact des membranes, ce qui conduit à des échanges et à des séparations améliorées. En outre, il n'y a pas lieu de régler la répartition du fluide traité (débit ou pression) entre les deux compartiments.

En se référant à la figure 1, on voit que l'appareil selon l'invention comporte au moins deux zones distinctes : une zone d'échanges (12) entre le fluide traité et un fluide auxiliaire et une zone de séparation (13) d'un composant du fluide traité. En effet, la zone (12) qui s'étend de part et d'autre de la membrane d'échanges (16) entre les compartiments (15) et (14) parcourus respectivement par le fluide traité et par le fluide auxiliaire constitue la zone d'échanges. Et la zone (13) qui s'étend de part et d'autre de la membrane de séparation (21) entre le compartiment (22) parcouru par le fluide traité et le compartiment (23) correspondant muni de moyens permettant l'évacuation d'au moins un composant du fluide traité est la zone de séparation.

Ces deux zones sont parcourues l'une et l'autre, d'un même côté de la membrane, par le fluide traité, soit simultanément par des circuits "en parallèle" (cf. figure 1), soit de préférence successivement dans l'espace, par des circuits "en série" (cf. figure 2). Dans ce dernier cas le fluide traité peut traverser d'abord la zone d'échanges

.../...

puis la zone de séparation, ou l'inverse. Le fluide traité peut traverser l'appareil une seule fois, ou plusieurs fois, lorsqu'il est recyclé, soit en partie, soit en totalité.

Avantageusement des moyens de types connus en soi, par exemple un diaphragme règlable ou une pince peuvent équiper une canalisation flexible, pour permettre de règler la section de passage offerte au fluide traité passant d'un compartiment à l'autre. On dispose ainsi d'un moyen de règlage de la pression du fluide traité dans la zone de séparation, donc d'un moyen pratique d'action sur la séparation elle-même.

La séparation peut d'ailleurs être règlée aussi soit par dépression appliquée sur le deuxième compartiment (23) dans la zone de séparation, par exemple à l'aide d'une pompe, soit par gravité, soit par une combinaison de ces divers moyens.

Les opérations d'échanges et de séparation peuvent ainsi être conduites non seulement dans des zones distinctes, séparées, mais encore de façon pratiquement indépendante.

La zone d'échange (12) et la zone de séparation (13) sont équipées chacune d'une membrane respectivement (16) et (21). Généralement ces membranes sont semi-perméables, c'est-à-dire qu'elles permettent des échanges de matières sélectifs.

Des membranes microporeuses par exemple conviennent bien pour l'oxygénation du sang. Cependant la zone d'échanges peut être équipée d'une membrane étanche aux fluides, mais permettant des échanges de calories entre fluides. La zone d'échanges est alors utilisable en zone de chauffage, de refroidissement ou de régulation thermique du fluide traité.

Lorsque les membranes sont semi-perméables dans chacune des deux zones, elles peuvent être soit identiques et posséder les mêmes propriétés, soit au contraire de nature semblable ou différente et posséder des propriétés différentes, plus particulièrement adaptées à leurs fonctions propres respectivement d'échanges ou de séparation.

L'importance relative des zones d'échanges et de séparation dépend essentiellement des surfaces de membranes nécessaires pour mettre en oeuvre séparément ces deux fonctions. Elle dépend donc des caractéristiques propres des membranes et des performances demandées en fonction de l'application envisagée. Généralement elle n'est pas critique.

La zone d'échanges (12) peut être parcourue par le fluide traité

.../...

et par le fluide auxiliaire, de part et d'autre de la membrane d'échanges (16), soit à contre-courant, soit à co-courant. Cette dernière disposition présente ici l'avantage de permettre d'abaisser la différence de pression moyenne pouvant exister entre le fluide traité et le fluide auxiliaire et ainsi de limiter l'effet secondaire de séparation pouvant éventuellement se produire au niveau de la membrane dans la zone d'échanges.

Le compartiment (22) de l'appareil selon la figure 1 est muni de deux orifices (24) et (25) permettant respectivement l'introduction et l'évacuation du fluide traité. Le compartiment (23) est muni d'un orifice (26) permettant l'évacuation de la fraction du fluide traité séparée du fluide traité à travers la membrane (21). Avantageusement un orifice auxiliaire (27) permet la circulation d'un fluide dans le compartiment (23), par exemple d'un fluide ayant un rôle de nettoyage ou de stérilisation, préalablement à l'usage de l'appareil.

Les compartiments (15) et (22) parcourus par le fluide traité forment la première région limitée par les membranes (16) et (21). La seconde région est constituée par les deux compartiments extérieurs (14) et (23) qui, selon l'invention, sont des compartiments distincts, totalement isolés l'un de l'autre, de manière étanche aux fluides. Ils sont en effet séparés l'un de l'autre d'une part par la première région, d'autre part par l'enveloppe (10), étanche aux fluides.

Comme on le constate, chacune des deux zones (12) et (13) de l'appareil selon la figure 1 est parcourue simultanément en parallèle par le fluide traité. Chacune des deux zones (12) et (13) peut fonctionner pratiquement indépendamment l'une de l'autre. Les conditions de débit, de vitesses moyennes d'écoulement du fluide traité au contact de chacune des membranes, les pressions du fluide traité au contact de chacune des membranes, les pressions du fluide traité à l'intérieur de chaque zone peuvent être réglées et contrôlées indépendamment grâce à l'aide de tous moyens extérieurs connus tels que pompes, vannes, diaphragmes, manomètres, débitmètres, etc ...

Avantageusement on peut relier entre eux les orifices (18) et (25) par une canalisation (non représentée). On obtiendra alors une circulation "en série" du fluide traité dans la zone (12) puis dans la zone (13). On obtient ainsi un appareil semblable à celui représenté figure 2. Celui-ci ne diffère en effet de l'appareil représenté figure 1 que par le fait qu'il comporte des moyens de communication interne (28)

...../....

8

permettant au fluide traité de passer de la zone (12), après l'avoir traversée, à la zone (13), sans sortir à l'extérieur de l'appareil.

Un mode de réalisation particulier de l'invention est représenté figure 3. L'appareil selon la figure 3 se distingue de l'appareil représenté figure 1 essentiellement par l'absence de cloison (11) ; la première région se confond alors avec les compartiments (15) et (22). Les zones d'échanges et de séparation sont distinctes et séparées par une cloison virtuelle équidistante des membranes d'échanges et de séparation.

Elles ne sont pas indépendantes car d'une part, les compartiments (15) et (22) parcourus par le fluide à traiter sont en commun. D'autre part, la pression, la direction de l'écoulement et les vitesses du fluide traité étant sensiblement les mêmes au contact de chacune des membranes (16) et (21), le fonctionnement de la zone de séparation, au niveau de la membrane (21) n'est pas totalement indépendant des conditions de fonctionnement de la zone d'échanges au niveau de la membrane (16) et réciproquement.

Mais ces zones sont distinctes, car la zone de séparation fonctionne comme dans l'appareil représenté figure 1, généralement à l'exclusion de tout échange et la zone d'échanges fonctionne également comme selon l'appareil représenté figure 1, séparément de la zone de séparation.

Un appareil selon la figure 3 peut être pratiquement construit par la superposition de jeux de trois compartiments semblables à ceux représentés, répétée autant de fois que nécessaire, les orifices homologues étant raccordés entre eux de manière connue en soi. Des intercalaires (non représentés) constitués par exemple de grilles, peuvent avantageusement à la fois soutenir les membranes et permettre l'écoulement du fluide auxiliaire dans le compartiment correspondant.

Dans un tel assemblage, les compartiments (15) et (22) traversés par le fluide traité peuvent, en variante, être reliés en série à un ou à plusieurs compartiments homologues.

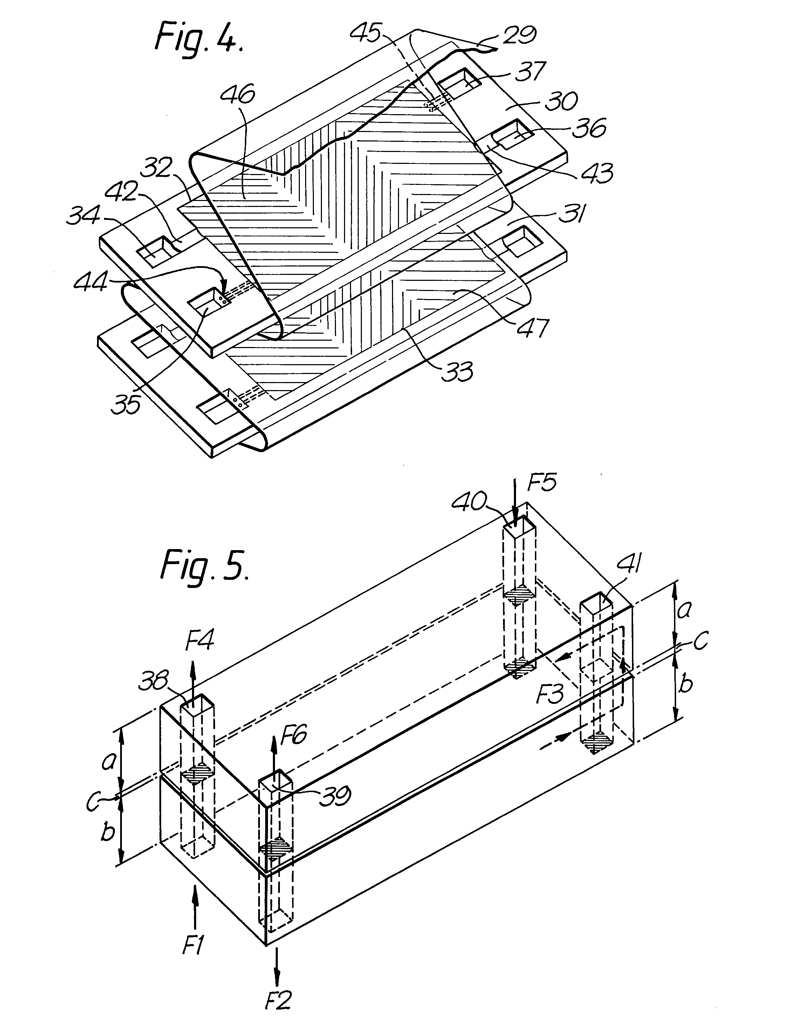

Les figures 4 et 5 représentent respectivement une vue de détail partielle et une vue d'ensemble en perspective d'un mode de réalisation particulier de l'appareil selon l'invention tel que représenté schématiquement figure 2.

Un tel mode de réalisation est constitué par l'empilement alterné de membranes et d'intercalaires de formes générales planes, rectangulaires. Une première zone est constituée par l'empilement d'intercalaires alternés avec une première membrane pliée en zig-zag et
.../...

s'étend selon une hauteur a. Une deuxième zone constituée par un empilement semblable d'intercalaires alternant avec une deuxième membrane pliée en zig-zag s'étend sur une hauteur b. Les deux zones sont séparées par un intercalaire spécial c.

L'ensemble des éléments constituants les zones a et b et l'intercalaire c est disposé de manière connue en soi, entre deux plaques parallèles, non représentées pour la clarté du dessin. Ces plaques maintiennent ces éléments serrés de façon étanche, grâce par exemple à une série de boulons les reliant.

La zone (b) est la zone de séparation et la zone (a) est la zone d'échanges. Le détail de la structure d'une zone telle que (a) ou (b) apparaît figure 4. La membrane (29) est pliée en zig-zag entre des intercalaires identiques (30) et (31). Chaque intercalaire est d'un type connu en soi, constitué par exemple par une feuille flexible formée par injection de matière thermoplastique. Il comporte de préférence sur ces deux faces une zone centrale à l'intérieur d'un cadre (32) ou (33) délimitant à l'intérieur soit la zone d'échanges, soit la zone de séparation.

La zone centrale est constituée par une partie amincie de la feuille de matière plastique munie d'éléments en relief formant des nervures (46), (47), des butées d'écartement ou des multipoints. La membrane recouvre entièrement la zone centrale de chaque intercalaire et repose sur les éléments en relief. Ainsi le fluide traité peut se déplacer sous forme de film mince entre les deux faces d'un pli de la membrane et le fluide auxiliaire peut se déplacer dans les espaces ménagés entre une face de l'intercalaire et la membrane reposant sur cette face.

Chaque angle de l'intercalaire est muni d'une ouverture telle que (34), (35), (36), (37). Par superposition des intercalaires, ces ouvertures forment des canaux ou puits de distribution de fluides tels que (38), (39), (40), (41). Chaque ouverture communique avec ladite zone centrale, soit par des canaux ouverts tels que (42), (43), soit par des conduits internes tels que (44), (45). Les canaux (42), (43) sur les parois desquels repose la membrane (29) mettent en communication les ouvertures diagonalement opposées (34), (36) avec l'espace compris dans le pli de la membrane. Les conduits internes (44), (45) mettent en communication les ouvertures diagonalement opposées (35), (37) avec l'espace compris entre l'intercalaire correspondant et la membrane reposant sur cet intercalaire.

.../...

10

Des moyens d'étanchéité connus en soi (non représentés) tels que des nervures encadrent les ouvertures (34), (35), (36), (37) de sorte que l'étanchéité est réalisée par serrage des membranes et des intercalaires.

La plaque c se distingue essentiellement des intercalaires ordinaires tels que (30) et (31) par le fait qu'elle ne possède qu'une seule ouverture (telle que 36) pour le passage du fluide traité d'une zone à l'autre. Les trois autres ouvertures peuvent exister dans la plaque c, mais elles doivent alors être bouchées de manière étanche par tous moyens connus tels que des pièces rapportées et scellées (zones hâchurées).

L'appareil représenté figures 4 et 5 fonctionne de la manière suivante : le fluide à traiter pénètre à la base du puits (38) dans la zone b selon la flèche $F_1$, de là il se répartit en parallèle par des canaux latéraux superposés tels que (34) et il s'étale à chaque niveau en film mince à l'intérieur des plis de la membrane sur la surface de la zone de séparation délimitée par un cadre tel que (32). Puis, après séparation d'un constituant qui est évacué par des conduits internes tels que (44) vers la partie inférieure du puits (39), flèche $F_2$, il gagne le puits (41) par des canaux latéraux tels que (43), flèche $F_3$.

Passant de la zone b à la zone a il effectue un parcours semblable en direction générale opposée et est évacué à la partie supérieure du puits (38), flèche $F_4$. Un fluide auxiliaire pénètre par le puits (40), flèche $F_5$ et se répartit en parallèle à différents niveaux par des canaux latéraux superposés tels que (45) dans la zone d'échanges comprise entre les intercalaires et la membrane repliée en zig-zag dans la zone a. Ce fluide se rassemble après échanges par des canaux tels que (44) dans la partie supérieure du puits (39) d'où il est évacué, flèche $F_6$.

L'appareil représenté figure 5 peut être équipé d'intercalaires de tous types connus, notamment sous forme de grilles. Les membranes peuvent être constituées d'éléments pliés chacun autour ou à l'intérieur d'un intercalaire. Elles peuvent aussi être constituées de simples feuilles posées à plat sur la zone d'échanges ou encore être assemblées par paires et scellées sur leur pourtour à l'exception de deux endroits opposés correspondant aux canaux latéraux tels que (42), (43).

L'appareil représenté figures 4 et 5 peut ne comporter qu'une seule membrane pliée en zig-zag, d'une extrémité à l'autre de l'appareil, entre les différents intercalaires, y compris la plaque c.

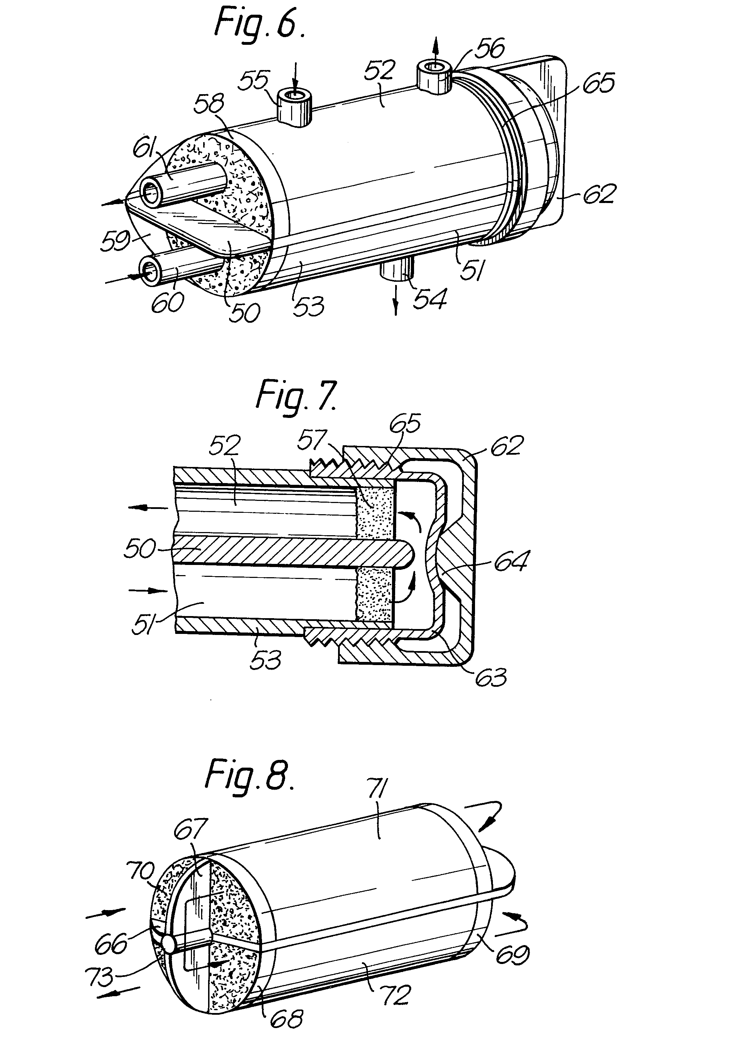

L'appareil représenté figure 6 est équipé de membranes se présentant sous forme de fibres creuses. Ces fibres creuses ont une
.../...

forme générale tubulaire. Leur diamètre extérieur est généralement inférieur à 1 mm, de préférence inférieur à 0,6 mm et généralement supérieur à 5 µ, de préférence supérieur à 200 µ. Elles peuvent être de tous types connus et en tout matériau convenable connu, choisi en fonction de l'application envisagée.

Sans que cette disposition soit critique, les fibres creuses sont généralement groupées en faisceaux de fibres disposées sensiblement parallèlement les unes aux autres. Elles peuvent présenter localement des inclinaisons relatives les unes par rapport aux autres, par exemple être torsadées entre elles. Leurs extrémités, généralement ouvertes, sont groupées, souvent le long d'un plan perpendiculaire à l'axe du faisceau.

Selon la figure 6, une plaque (50) sépare deux faisceaux parallèles de fibres creuses (51) et (52), l'ensemble étant logé dans un boîtier cylindrique transparent (53) muni d'orifices (54) pour l'évacuation d'un constituant du fluide traité, (55) et (56) pour l'introduction et l'évacuation d'un fluide auxiliaire. Les fibres creuses sont scellées de manière étanche à l'extérieur, d'une part entres elles, vers leurs extrémités ouvertes et d'autre part au boîtier (53), dans les zones (57) et (58) à l'aide d'une résine polymérisable.

L'extrémité avant de l'appareil est équipée d'un couvercle arrondi (59) muni d'orifices (60) et (61) pour l'introduction et l'évacuation du fluide traité. Ce couvercle prend appui de façon étanche sur le bord de la plaque (50). Un couvercle (62) ferme l'extrémité arrière de l'appareil ; un espace est ménagé entre le couvercle arrière et la plaque (50).

Le fluide traité pénètre par l'orifice (60) dans l'appareil, puis à l'intérieur des fibres creuses du faisceau (51). A l'arrière de l'appareil il contourne la plaque (50) à l'intérieur du couvercle (62), puis pénètre dans les fibres creuses du faisceau (52) pour sortir par l'orifice (61) après avoir subi séparément un traitement de séparation puis un traitement d'échanges avec le fluide auxiliaire qui se déplace à l'extérieur des fibres creuses du faisceau (52).

La figure 7 représente une variante de réalisation de la partie arrière de l'appareil selon la figure 6. Les éléments homologues sont désignés par les mêmes numéros. Le couvercle arrière est constitué d'une part d'une cuvette (63) à paroi mince et flexible et d'autre part d'un bouchon (62), épais, muni d'un bossage central interne (64). Les bords de la cuvette (63) sont emmanchés sur le boîtier (53), ils comportent extérieurement un filetage (65) sur lequel se visse le bouchon (62).

.../...

En vissant le bouchon (62) on diminue le passage du fluide traité en regard de la plaque fixe (50), ou inversement. On peut ainsi règler le débit ou de préférence la pression du fluide traité en amont, c'est-à-dire dans la zone de séparation constituée par le faisceau (51).

La figure 8 représente un ensemble de quatre faisceaux de fibres creuses disposées selon des axes parallèles, séparés par deux cloisons perpendiculaires (66) et (67). Les zones d'empotage réalisant l'étanchéité entre les fibres creuses à leurs extrémités sont dans des plans parallèles (68), (69) perpendiculaires à l'axe des faisceaux. Pour simplifier le dessin, les couvercles et les divers orifices de l'appareil ne sont pas représentés. La découpe des cloisons permet au fluide traité de parcourir successivement l'intérieur des faisceaux (70), (71), (72) et (73). Un tel appareil permet ainsi de réaliser séparément sur un circuit extracorporel de sang, quatre traitements distincts, par exemple l'ultrafiltration, la dialyse, l'oxygénation et le réchauffage du sang. On peut réserver en effet trois sous-compartiments pour l'exercice d'une même fonction d'échanges avec des fluides auxiliaires distincts.

Un appareil de ce type permet aussi d'offrir instantanément des surfaces de membranes plus ou moins importantes pour telle ou telle fonction selon l'application envisagée.

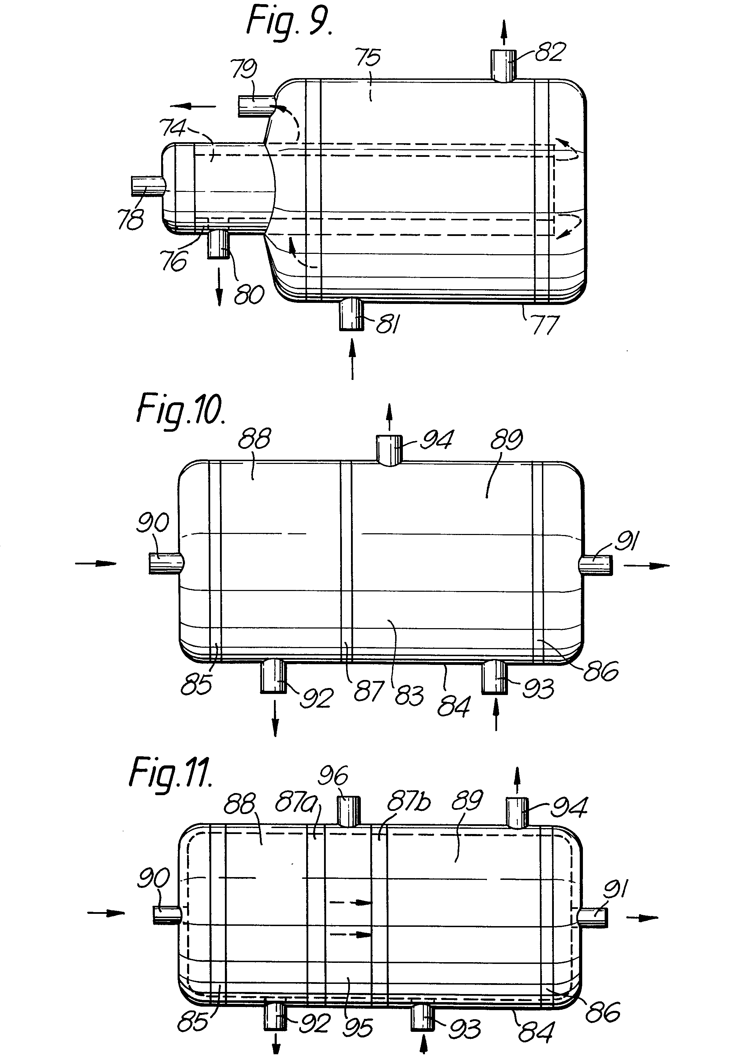

La figure 9 représente un appareil équipé de deux faisceaux de fibres creuses coaxiaux (74) et (75) ouvertes à leurs extrémités. Le faisceau (74) est disposé à l'intérieur d'une enveloppe cylindrique (76) qui le sépare de façon étanche du faisceau (75), lequel est logé à l'intérieur d'une enveloppe cylindrique coaxiale (77). Le fluide à traiter pénètre par l'orifice (78) à l'intérieur des fibres creuses du faisceau (74), revient en traversant les fibres creuses du faisceau (75) et est évacué par l'orifice (79). Le fluide séparé du fluide traité est évacué par l'orifice (80). Un fluide auxiliaire circule à l'extérieur des fibres creuses du faisceau (75), après avoir pénétré par l'orifice (81) et avant de sortir par l'orifice (82).

En variante, on peut remplacer le faisceau extérieur (75) par une membrane tubulaire aplatie roulée avec un intercalaire convenable en spirale autour du mandrin constitué par l'enveloppe cylindrique du faisceau de fibres creuses (74). Avantageusement l'axe de l'appareil est vertical. Une canalisation appropriée relie en série l'extrémité du faisceau (74) {à la sortie des fibres creuses} constituant la zone de séparation, à une extrémité de la membrane tubulaire aplatie constituant la zone d'échanges. Un tel appareil peut comporter par ailleurs toutes les dispositions connues des appareils d'échanges à membrane tubulaire aplatie roulée en spirale.

La figure 10 représente un appareil équipé d'un faisceau sensiblement cylindrique de fibres creuses (83) logé à l'intérieur d'une enveloppe (84). Les fibres creuses sont scellées à leurs extrémités (85) et (86) de manière étanche à l'aide d'un produit d'empotage tel qu'une résine polymérisable. Au cours du processus de fabrication du faisceau un tel produit est également introduit entre les deux extrémités en (87). Il forme ainsi une cloison étanche séparant l'appareil en deux zones distinctes (88) et (89). Le fluide traité pénètre par l'orifice (90), traverse les fibres creuses, puis sort par l'orifice (91). Le composant séparé du fluide traité sort par l'orifice (92). Le fluide auxiliaire pénètre à l'extérieur des fibres creuses par l'orifice (93) et sort par l'orifice (94).

La figure 11 représente un appareil qui se distingue de l'appareil selon la figure 10 essentiellement par le fait qu'il comporte deux faisceaux coaxiaux indépendants (95) et (96) que l'on peut obtenir par exemple en tranchant le faisceau (83) dans la zone d'empotage intermédiaire (87). Les mêmes numéros désignent les éléments homologues des deux appareils. La zone d'empotage (87) constituant une cloison séparant deux compartiments se dédouble donc en deux cloisons étanches se faisant face (87a) et (87b). On forme ainsi entre les deux zones (88) et (89) un espace intermédiaire (95) qui peut, le cas échéant, être muni d'un orifice (96) pour l'introduction ou l'évacuation de fluide.

L'appareil selon l'invention peut donc comporter dans sa réalisation toutes les dispositions connues des appareils à membranes, quel que soit leurs types, à membrane plane, roulée en spirale ou sous forme de fibres creuses. Il peut combiner éventuellement les caractéristiques de deux de ces types d'appareils. Il peut faire l'objet de diverses autres variantes d'exécution à la portée du technicien.

L'appareil selon l'invention peut faire l'objet de nombreuses applications dans le cadre du Génie Médical. Son emploi peut être envisagé chaque fois qu'un échange de matières et/ou de calories doit être effectué en même temps que la séparation de constituants d'un fluide, liquide ou gazeux, notamment un fluide biologique.

L'efficacité de l'appareil selon l'invention est d'autant plus grande que l'on sépare mieux en pratique les fonctions d'échanges et de séparation, c'est-à-dire qu'on les rend pratiquement indépendantes. Il peut être avantageux pour cela de réduire les pertes de charge entre

.../...

les extrémités d'une même région ou d'un même compartiment par exemple par un dessin approprié des intercalaires soutenant la membrane. On a intérêt en outre à maîtriser le mieux possible et éventuellement à réduire la différence de pression pouvant exister de part et d'autre de la membrane dans la zone d'échanges.

Il est avantageux pour cela de disposer sur le circuit du fluide traité, par exemple le sang, l'appareil selon l'invention et de l'intercaler en série entre deux pompes péristaltiques capables de maintenir automatiquement la pression du sang à l'intérieur dudit appareil comprise entre des valeurs prédéterminées. Les pompes décrites dans le brevet français enregistré sous le numéro 74/39 848 conviennent particulièrement bien à cet effet.

Par ailleurs la séparation d'un constituant du fluide traité peut avantageusement être contrôlée et commandée de façon prédéterminée par la mise en oeuvre de l'une des techniques décrites dans les brevets français enregistrés sous les numéros 71/43 720, 72/27 582 ou 76/39 836.

L'appareil selon l'invention est tout spécialement intéressant pour le traitement extracorporel de sang. Par exemple certains patients sont atteints de surcharge hydrique au point d'être affectés d'un oedème aigu du poumon, lequel entraîne une insuffisance respiratoire. L'appareil selon l'invention permet alors d'assurer simultanément et séparément dans les meilleures conditions d'économie, de sécurité et d'efficacité la double fonction d'oxygénation de sang et d'ultrafiltration du sang en éliminant l'excès d'eau. Il permet ainsi de rétablir à la fin d'un traitement d'oxygénation du sang l'hématocrite initiale par concentration du sang (élimination par ultrafiltration de l'eau en excès).

C'est plus particulièrement dans le domaine de l'épuration du sang par dialyse et ultrafiltration que l'appareil selon l'invention trouve de nombreuses applications. Il permet en effet d'exercer simultanément et séparément, donc indépendamment, les fonctions de dialyse et d'ultrafiltration du sang. Il offre ainsi de façon sûre et économique une souplesse d'emploi inconnue jusqu'à présent pour la mise en oeuvre des nombreuses stratégies possibles d'épuration du sang dont plusieurs sont déjà utilisées.

C'est ainsi qu'il permet des traitements par hémodialyse et ultrafiltration simultanées selon des taux constants ou variables, indépendamment l'un par rapport à l'autre. Il permet également des traitements séquentiels par hémodialyse et ultrafiltration alternés ou

.../...

se chevauchant et selon les fréquences désirées. Il permet aussi des traitements par hémofiltration et hémodialyse combinés. Il permet encore de réchauffer le sang au cours d'une séance d'ultrafiltration ou d'hémofiltration.

On peut désirer, pour des raisons de sécurité, maintenir le sang à une pression sensiblement plus élevée que celle du liquide de dialyse. On peut alors, par exemple à l'aide des deux pompes péristaltiques mentionnées précédemment, maîtriser cette pression transmembranaire, c'est-à-dire la maintenir entre des limites prédéterminées et ainsi être en mesure d'estimer avec précision le débit et/ou le volume d'ultrafiltration correspondant qu'il y aura lieu d'ajouter au débit et/ou au volume d'ultrafiltrat mesuré pour connaître à tout moment avec une bonne précision le débit et/ou le volume total d'ultrafiltrat soutiré au patient.

L'appareil selon l'invention présente de nombreux avantages. Il remplace à lui seul deux appareils homologues, car il assure séparément et souvent de façon pratiquement indépendante deux fonctions distinctes. D'où une économie sensible notamment sur du matériel à usage unique, une mise en oeuvre plus aisée et plus sûre et, dans le cas de circulation extracorporelle de sang, un faible volume de sang extravasé hors du patient. Il offre une grande souplesse d'emploi, permettant d'exécuter avec précision de nombreuses stratégies de traitements. Il permet de retirer un ultrafiltrat pur et non mélangé à un liquide auxiliaire. Il est alors aisé de procéder à une ou à plusieurs analyses successives de cet ultrafiltrat ce qui peut permettre d'orienter avec précision et efficacité la suite du traitement. Par ailleurs, le fluide traité, circulant en série et non en parallèle dans des compartiments distincts, a une vitesse de circulation plus élevée à débit égal, ce qui contribue à l'efficacité des traitements.

REVENDICATIONS

1 - Appareil pour le traitement de fluides en Génie Médical, comprenant une enveloppe munie d'orifices pour l'introduction et l'évacuation de fluides, caractérisé en ce qu'il comprend au moins une membrane séparant ledit appareil en une première région parcourue par le fluide traité et une deuxième région, ladite deuxième région comportant des moyens la divisant de manière étanche aux fluides en deux compartiments distincts, le premier compartiment étant muni de moyens permettant la circulation d'un fluide auxiliaire et le deuxième compartiment étant muni de moyens permettant l'évacuation d'au moins un composant du fluide traité.

2 - Appareil selon la revendication 1, caractérisé en ce que l'un au moins desdits compartiments de la deuxième région est subdivisé en au moins deux sous-compartiments, chaque sous-compartiment étant muni de moyens lui permettant d'assurer séparément une fonction de même nature, échanges ou séparation, que le compartiment auquel il appartient.

3 - Appareil selon l'une quelconque des revendications précédentes, caractérisé en ce que ladite première région comporte des moyens la séparant de manière étanche aux fluides en au moins deux compartiments distincts.

4 - Appareil selon la revendication 3, caractérisé en ce que à chaque compartiment de ladite deuxième région correspond un compartiment de ladite première région, la zone s'étendant de part et d'autre de ladite membrane entre les compartiments parcourus par le fluide traité et par le fluide auxiliaire constituant une zone dite d'échanges et la zone s'étendant de part et d'autre de ladite membrane entre le compartiment parcouru par le fluide traité et le compartiment correspondant muni de moyens permettant l'évacuation d'au moins un composant du fluide traité, constituant une zone dite de séparation.

5 - Appareil selon la revendication 3, caractérisé en ce que lesdits moyens séparant l'une et/ou l'autre des régions en deux compartiments distincts sont constitués par une cloison s'étendant de manière étanche aux fluides entre ladite enveloppe et ladite membrane.

6 - Appareil selon la revendication 5, caractérisé en ce que l'une au moins desdites cloisons est dédoublée en deux cloisons parallèles ménageant entre elles un espace intermédiaire.

7 - Appareil selon l'une quelconque des revendications 3 à 6, caractérisé en ce que lesdits compartiments de ladite première région sont reliés en série par tout moyen, connu en soi, permettant l'écoulement de fluides.

8 - Appareil selon l'une quelconque des revendications précédentes, caractérisé en ce qu'une même membrane ou des membranes de mêmes caractéristiques, équipent lesdites zones d'échanges et de séparation.

9 - Appareil selon la revendication 4, comportant des moyens permettant au fluide traité de traverser d'abord la zone dite de séparation avant de traverser la zone dite d'échanges, caractérisé en ce qu'il comporte des moyens pour règler la pression du fluide traité dans la zone dite de séparation.

10 - Appareil selon l'une quelconque des revendications précédentes, caractérisé en ce qu'il appartient au type d'appareils comportant des intercalaires de forme générale plane constitués de grilles et/ou de minces plaques munies d'éléments en relief, une ou plusieurs membranes étant disposées entre lesdits intercalaires.

11 - Appareil selon l'une quelconque des revendications 1 à 9, caractérisé en ce qu'il appartient au type d'appareils dont la membrane est constituée de fibres creuses.

12 - Appareil selon la revendication 11, caractérisé en ce que chacune desdites zones comporte un faisceau de fibres creuses ouvertes à leurs extrémités, lesdits faisceaux étant disposés de part et d'autre d'une cloison étanche selon des axes sensiblement parallèles.

13 - Appareil selon la revendication 11, caractérisé en ce que lesdites zones d'échanges et de séparation sont constituées de faisceaux de fibres creuses coaxiaux.

14 - Application d'un appareil selon l'une quelconque des revendications précédentes au traitement extracorporel de sang.

15 - Application d'un appareil selon l'une quelconque des revendications 1 à 13 au traitement extracorporel de sang par hémodialyse et ultrafiltration simultanées et séparées.

16 - Circuit extracorporel de sang caractérisé en ce qu'il comprend un appareil selon l'une quelconque des revendications 1 à 13, deux pompes péristaltiques capables de maintenir automatiquement la pression du sang à l'intérieur dudit appareil compris entre des valeurs prédéterminées et des éléments de raccordement.

1|5

**Fig.1.**

**Fig.2.**

**Fig.3.**

0014166

215

Fig. 4.

Fig. 5.

0014166

Fig. 6.

315

Fig. 7.

Fig. 8.

Fig. 9.

415

79    75    82

74    78    76    80    81    77

Fig. 10.

88    94    89

90    91

85    92    87    83    84    93    86

Fig. 11.

88    87a    96    87b    89    94

90    91

85    92    95    93    84    86

## Fig.12.

## Fig.13.

**RAPPORT DE RECHERCHE EUROPEENNE**

Office européen
des brevets

Numéro de la demande
EP 80 42 0007

## DOCUMENTS CONSIDERES COMME PERTINENTS

| Catégorie | Citation du document avec indication, en cas de besoin, des parties pertinentes | Revendication concernée |
|---|---|---|
| X | FR - A - 2 127 155 (RHONE POULENC) <br> * Figures 11,12; page 6, lignes 4-18; page 1, lignes 20-22 * | 1,2,4, 14,15 |
| X | US - A - 3 998 593 (YOSHIDA) <br> * Figures 1,2; colonne 2, ligne 35 - colonne 3, ligne 15 * | 1,2,14 |
| | DE - A - 2 051 007 (LEONARD) <br> * Figure 1; page 8, lignes 1-18; page 16, lignes 8-23 * | 3,14 |
| | US - A - 3 416 664 (KUMME) <br> * Figure 1; colonne 1, lignes 24-39 * | 9,14 |
| | FR - A - 1 508 189 (BARNABE) <br> * Figures; page 6, lignes 15-39 * | 1,11, 12,14 |
| | FR - A - 2 354 116 (GAMBRO) <br> * Figure 9; page 5, ligne 35 - page 6, ligne 3 * | 9 |
| D | FR - A - 2 293 220 (RHONE POULENC) | 16 |
| D | FR - A - 2 163 906 (RHONE POULENC) | |
| D | FR - A - 2 193 624 (RHONE POULENC) | |
| D | FR - A - 2 336 961 (RHONE POULENC) | |

**CLASSEMENT DE LA DEMANDE (Int. Cl. ³)**

A 61 M 1/03

**DOMAINES TECHNIQUES RECHERCHES (Int. Cl. ³)**

A 61 M

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

☒ Le présent rapport de recherche a été établi pour toutes les revendications

| Lieu de la recherche | Date d'achevement de la recherche | Examinateur |
|---|---|---|
| La Haye | 18-04-1980 | STEENBAKKER |

OEB Form 1503.1    06.78